# EUROPÄISCHE PATENTSCHRIFT

(11) **EP 2 946 754 B1**
(45) Veröffentlichungstag und Bekanntmachung des Hinweises auf die Patenterteilung: **01.03.2017**
(21) Anmeldenummer: 15001538.6
(22) Anmeldetag: 21.05.2015
(51) Int. Cl.: A61F 13/00, A61F 13/02

(54) **PFLASTER MIT BIELASTISCHER RÜCKSCHICHT**
PATCH WITH BIELASTIC BACKING LAYER
PAVÉ DOTÉ D'UNE COUCHE EXTÉRIEURE BI-ÉLASTIQUE

(30) Priorität: 23.05.2014 DE 102014007650
(43) Veröffentlichungstag der Anmeldung: 25.11.2015
(73) Patentinhaber: AMW GmbH, 83627 Warngau (DE)
(72) Erfinder: Kaffl, Hubert, 83627 Warngau (DE)
(74) Vertreter: Beckord & Niedlich

(56) Entgegenhaltungen:
- EP-A1- 1 462 121
- DE-A1-102005 033 720
- DE-U1- 29 812 144
- US-A- 5 225 199

## Beschreibung

Die Erfindung betrifft ein Pflaster mit einer bielastischen Rückschicht.

Pflaster mit elastischer oder bielastischer Rückschicht gehören zum Stand der Technik. Beispielsweise beschreibt US 4 780 168 A eine Wundbandage mit einer planaren Streckungscharakteristik im Bereich von 0,5 bis 10 pounds/inch. Nach EP 1 009 393 B1 sind jedoch Materialien mit einer derartigen Dehnbarkeit nicht als Rückschicht für Pflaster geeignet. Entweder ist ihre Dehnbarkeit zu gering, so dass sich beim Tragen auf der Haut ein unangenehmes Fremdkörpergefühl einstellt und auch die Gefahr besteht, dass das Pflaster die Bewegungen der Haut nicht ausreichend mitmacht und sich ablösen kann; EP 1 009 393 B1 (Vergleichsbeispiel 2). Sind die Materialien jedoch sehr dehnbar, so tritt der sogenannte Schüsselbildungs-Effekt auf; EP 1 009 393 B1 (Vergleichsbeispiel 1). Denn bei der Pflasterherstellung wird zuerst ein bandförmiger Verbund aus bzw. mit den Materialien für Rückschicht, Matrix-Schicht und Schutzschicht hergestellt. Dabei kann das Material der Schutzschicht weniger elastisch sein als das Material der Rückschicht. Wird das bandförmige Material der Rückschicht auf das bandförmige Material der Schutzschicht derart aufgebracht, dass beide Bänder die Matrix-Schicht einschließen, so ist das bandförmige Material der Rückschicht infolge angelegter Spannung stärker gestreckt als das Material der Schutzschicht. Wird nun das erhaltene Laminat einem Stanzvorgang unterworfen, um auf dem Band des Schutzschicht-Materials aus dem Band des Rückschicht-Materials einzelne Pflaster zu stanzen, führt die nach dem Stanzen wirkende elastische Rückstellkraft dazu, dass sich in Bandrichtung gegenüberliegende Randbereiche der vereinzelten Pflaster zurückziehen, während quer zur Bandrichtung gegenüberliegende Randbereiche sich nach außen entspannen bzw. ausdehnen können, wobei sich die vereinzelten Pflaster unter Bildung einer Schüssel mehr oder minder aufwerfen können (EP 1 009 393 B1 Vergleichsbeispiel 1). Dieser unerwünschte Effekt ist umso ausgeprägter, je größer die herzustellenden Produkte sind. Insbesondere längliche Produkte fallen dann nicht plan an, sondern wölben sich infolge der in Längs- und Querrichtung unterschiedlichen Spannungen und Rückstellkräfte bananenförmig auf, was eine Weiterverarbeitung erschwert oder gar unmöglich macht.

Diese Wanderungen der Randbereiche lassen sich daran erkennen, dass sich die in Bandrichtung gegenüberliegenden Randbereiche jeder Rückschicht hinter die Stanzspur zurückziehen, die das Stanzwerkzeug im Material der Schutzschicht hinterlässt, und dass sich die in Querrichtung gegenüberliegenden Randbereiche über die Stanzspur hinaus schieben und sie verdecken können. Beim Stanzvorgang muss sichergestellt sein, dass die Rückschichten der einzelnen Pflaster einwandfrei aus dem bandförmigen Rückschicht-Material herausgestanzt werden, wobei das Stanzwerkzeug leicht in das bandförmige Schutzschicht-Material eindringt und dort eine die Rückschicht zumindest im Moment des Stanzvorgangs eng umlaufende Spur hinterlässt. Dieses Eindringen des Stanzwerkzeugs in die Schutzschicht ist für die mechanischen Eigenschaften der Schutzschicht vernachlässigbar, führt also nicht dazu, dass die Schutzschicht beim späteren Abtrennen an der Spur reißt.

So schlägt EP 1 009 393 B1 ein Pflaster mit einer wiederablösbaren Schutzschicht, einer haftklebenden Reservoirschicht und einer gegebenenfalls haftkleberbeschichteten Rückschicht aus einem unidirektional und vorzugsweise längselastischen Material vor, das eine Elastizität von mindestens 20 % aufweist.

Ein Pflaster mit einer Rückschicht mit planarer Streckungscharakteristik oder mit einer bielastischen Rückschicht ist jedoch insofern vorzuziehen, als es den Bewegungen der Haut besser folgt.

Die Erfindung betrifft nun ein Pflaster
(a) mit einer obligatorischen gegebenenfalls haftkleber-beschichteten bielastischen Rückschicht (Overtape),
(b) mit einer obligatorischen Deckschicht,
(c1) mit einer haftklebenden Matrix-Schicht und einer fakultativen haftklebenden Klebemittel-Schicht oder
(c2) mit einer nicht-haftklebenden Matrix-Schicht und einer haftklebenden Klebemittel-Schicht und
(d) mit einer obligatorischen wiederablösbaren Schutzschicht, wobei
   - die Rückschicht ein bielastisches Gewebe oder ein bielastisches Flechtwerk (bielastisches Geflecht) oder ein bielastisches Gewirke umfasst oder daraus besteht,
   - die Bielastizität der Rückschicht dadurch gekennzeichnet ist, dass die Rückschicht in zwei verschiedenen Richtungen in nicht nur unwesentlichem Umfang elastisches Verhalten zeigt,
   wobei
   - bei einer Rückschicht mit oder aus einem Gewebe die beiden voneinander verschiedenen Richtungen weder parallel zur Kette des Gewebes noch in Richtung senkrecht zur Kette verlaufen und
   - bei einer Rückschicht mit oder aus einem Flechtwerk die beiden voneinander verschiedenen Richtungen weder parallel zum Strang des Flechtwerks noch in Richtung senkrecht zum Flechtstrang verlaufen und
   - bei einer Rückschicht mit oder aus einem Gewirke die beiden voneinander verschiedenen Richtungen weder parallel zur Richtung der Gewirkefäden noch in dazu senkrechter Richtung verlaufen.

Da derartige erfindungsgemäße Pflaster eine Rückschicht mit einer erfindungsgemäß gerichteten Bielastizität aufweisen, kann für die Herstellung der Pflaster ein Band aus dem bielastischen Rückschicht-Material eingesetzt werden, ohne dass die aus dem Band separierten einzelnen Rückschichten schrumpfen, so dass der Schüsselbildungs-Effekt nicht auftritt. Durch die Bielastizität ist jedoch Tragekomfort gewährleistet.

Ebenso betrifft die Erfindung ein Pflaster
(a) mit einer obligatorischen gegebenenfalls haftkleber-beschichteten bielastischen Rückschicht,
(b1) mit einer haftklebenden Matrix-Schicht und einer fakultativen haftklebenden Klebemittel-Schicht oder
(b2) mit einer nicht-haftklebenden Matrix-Schicht und einer haftklebenden Klebemittel-Schicht und
(c) mit einer obligatorischen wiederablösbaren Schutzschicht,
wobei die Bielastizität der bielastischen Rückschicht dadurch gekennzeichnet ist, dass die Rückschicht in zwei verschiedenen Richtungen in nicht nur unwesentlichem Umfang elastisches Verhalten zeigt, wobei
- die Rückschicht ein bielastisches Gewebe oder ein bielastisches Flechtwerk (bielastisches Geflecht) oder ein bielastisches Gewirke umfasst oder daraus besteht,
- die Bielastizität der Rückschicht dadurch gekennzeichnet ist, dass die Rückschicht in zwei verschiedenen Richtungen in nicht nur unwesentlichem Umfang elastisches Verhalten zeigt,
wobei
- bei einer Rückschicht mit oder aus einem Gewebe diese beiden voneinander verschiedenen Richtungen weder parallel zur Kette des Gewebes noch in Richtung senkrecht zur Kette verlaufen und
- bei einer Rückschicht mit oder aus einem Flechtwerk diese beiden voneinander verschiedenen Richtungen weder parallel zum Strang des Flechtwerks noch in Richtung senkrecht zum Flechtstrang verlaufen und
- bei einer Rückschicht mit oder aus einem Gewirke die beiden voneinander verschiedenen Richtungen weder parallel zur Richtung der Gewirkefäden noch in dazu senkrechter Richtung verlaufen.

Da derartige erfindungsgemäße Pflaster wiederum eine Rückschicht mit einer erfindungsgemäß gerichteten Bielastizität aufweisen, kann für die Herstellung der Pflaster ein Band aus dem bielastischen Rückschicht-Material eingesetzt werden, ohne dass die aus dem Band separierten einzelnen Rückschichten schrumpfen, so dass der Schüsselbildungs-Effekt nicht auftritt. Durch die Bielastizität ist jedoch Tragekomfort gewährleistet.

Bei einem Gewirk kann die Richtung der Gewirkefäden auch mit den Maschenstäbchen benachbarter Fäden beschrieben werden.

Eine Rückschicht ist insbesondere dann bielastisch, wenn sich ihre Reißdehnung in zwei verschiedenen Richtungen um nicht mehr als einen Faktor 2 unterscheidet und wenn sich ihr Elestizitätsmodul in zwei verschiedenen Richtungen um nicht mehr als einen Faktor 2 unterscheidet.

Eine Rückschicht zeigt in nicht nur unwesentlichem Umfang elastisches Verhalten, wenn ihre Reißdehnung mindestens 30 Prozent, vorzugsweise mehr als 30 Prozent und insbesondere mindestens 60 Prozent beträgt und/oder wenn nach einer Dehnung der Rückschicht auf 12/10 (zwölf Zehntel) ihrer ursprünglichen linearen Abmessung und Wegfall der dehnenden Kraft bzw. Spannung höchstens 11/10 (elf Zehntel) ihrer ursprünglichen linearen Abmessung verbleiben.

Beispielsweise gemäß EP 1 009 393 B1 ([0012] und [0013]) lässt sich Elastizität nach DIN 60 000, DIN 61 632 (April 1985) oder DIN 61 632 (Dezember 2009) messen.

In DIN 61 632:1985-04 (DIN 61 632 in der Fassung von April 1985) oder DIN 61 632:2009-12 (DIN 61 632 in der Fassung von Dezember 2009) ist eine Messvorschrift zur Bestimmung der Dehnung epsilon_ges für Idealbinden angegeben, die auch auf die erfindungsgemäß verwendete bielastische Rückschicht anwendbar ist.

Die gemäß DIN 61 632:1985-04 oder DIN 61 632:2009-12 gemessene Dehnung epsilon_ges der bielastischen Rückschicht sollte in zwei voneinander verschiedenen Richtungen mindestens 10 % betragen. Eine Dehnung bzw. Dehnbarkeit von weniger als 5 % wird als unelastisch angesehen. Die Dehnung epsilon_ges kann in den zwei voneinander verschiedenen Richtungen dieselbe sein. Die Dehnung epsilon_ges kann aber auch in den beiden voneinander verschiedenen Richtungen verschieden sein.

So kann die Dehnung epsilon_ges in mindestens einer dieser beiden voneinander verschiedenen Richtungen oder in beiden Richtungen mindestens 30 % betragen (jeweils gemessen nach DIN 61632).

So kann die Dehnung epsilon_ges der bielastischen Rückschicht ferner in mindestens einer der beiden voneinander verschiedenen Richtungen oder in beiden Richtungen 30 bis 56 %, besonders 35 bis 56 % betragen, insbesondere 35 bis 70 %, vorzugsweise 40 bis 80 %, besonders bevorzugt 44 bis 150 % und vor allem 56 bis 170 % (jeweils gemessen nach DIN 61632).

Bei einem erfindungsgemäßen Pflaster können die zwei voneinander verschiedenen Richtungen
- bei einer Rückschicht mit oder aus Gewebe sowohl zum Schuss als auch zur Richtung senkrecht dazu schräg oder geneigt sein und
- bei einer Rückschicht mit oder aus Flechtwerk sowohl zum Flechtstrang als auch zur Richtung senkrecht dazu schräg oder geneigt sein und
- bei einer Rückschicht mit oder aus Gewirke sowohl zur Richtung der Gewirkefäden als auch zur dazu senkrechten Richtng schräg oder geneigt sein.

In anderen Richtungen kann die bielastische Rückschicht unelastisches Verhalten zeigen.

Gegenüber der Kette eines Gewebes oder quer dazu kann diese Neigung für mindestens eine der beiden voneinander verschiedenen Richtungen bis 45 Grad betragen. So können die beiden voneinander verschiedenen Richtungen in Bezug auf die Kette und quer dazu diagonal verlaufen.

Gegenüber dem Strang eines Geflechts oder quer dazu kann diese Neigung für mindestens eine der beiden voneinander verschiedenen Richtungen bis 45 Grad betragen. So können die beiden voneinander verschiedenen Richtungen in Bezug auf den Strang und quer dazu diagonal verlaufen.

Gegenüber der Richtung der Fäden eines Gewirks oder quer dazu kann diese Neigung für mindestens eine der beiden voneinander verschiedenen Richtungen bis 45 Grad betragen. So können die beiden voneinander verschiedenen Richtungen in Bezug auf die Fäden und quer dazu diagonal verlaufen.

Bei diesen Ausführungsformen kann die Rückschicht in Richtung parallel und quer zur Kette eines Gewebes oder parallel und quer zum Strang eines Flechtwerks oder parallel und quer zu den Fäden eines Gewirks unelastisches Verhalten zeigen, während die Dehnung epsilon_ges in zwei anderen und voneinander unabhängigen Richtungen mindestens 10 % betragen kann, beispielsweise 35 bis 56 %, insbesondere 35 bis 70 %, vorzugsweise 40 bis 80 %, besonders bevorzugt 44 bis 150 % und vor allem 56 bis 170 % (jeweils gemessen nach DIN 61632). Bielastisches oder diagonal elastisches Gewebe gehört zum Stand der Technik und ist im Handel erhältlich. Es kann beispielsweise von der Fa. Karl Otto Braun (KOB) bezogen werden. Bielastisches oder diagonal elastisches Flechtwerk oder Gewirk kann in Analogie zu bielastischem oder diagonal elastischem Gewebe hergestellt werden.

Auch erfindungsgemäße Pflaster mit diesen Merkmalen bieten vollen Tragekomfort, da sie bielastisch sind.

Bei dem erfindungsgemäßen Pflaster kann die bielastische Rückschicht die Matrix-Schicht samt Deckschicht auf der Schutzschicht allseitig abdecken oder die bielastische Rückschicht kann die Matrix-Schicht auf der Schutzschicht bedecken oder allseitig abdecken.

Das erfindungsgemäße Pflaster kann ein Schutzpflaster, ein Wundpflaster, ein kosmetisches Pflaster oder ein transdermales und insbesondere therapeutisches Pflaster sein. Als kosmetisches Pflaster oder als transdermales und insbesondere therapeutisches Pflaster kann die Matrix-Schicht Wirkstoffe enthalten.

Das Material der bielastischen Rückschicht kann ein Faden- oder Filament-Material, beispielsweise ein bielastisches Gewebe oder oder es kann ein bielastisches Flechtwerk (Geflecht), das schichtförmig ausgebildet sein kann, oder es kann ein bielastisches Gewirk sein. Diese Materialien können miteinander, einem bielastischen textilen Stoff, einem bielastischen Vlies, einer gegebenenfalls offenporigen bielastischen Schaumfolie oder einer bielastischen Folie kombiniert sein. Beispiele für derartige Materialien sind EP 1 009 393 B1 ([0017]) zu entnehmen.

Die bielestische Rückschicht kann wirkstoffundurchlässig sein.

Das Gewebe oder Flechtwerk oder Gewirk der bielastischen Rückschicht kann aus einem Faden- oder Filament-Material aus Polyester oder Polyurethan bestehen, insbesondere Polyethylenterephthalat.

Das Gewebe oder Flechtwerk oder Gewirk der bielastische Rückschicht kann aus Polyethylenterephthalat eines Flächengewichts von 40 bis 150, besonders 60 bis 150, insbesondere 70 bis 140, vorzugsweise 100 bis 140 und besonders bevorzugt 120 bis 140 g/m² bestehen.

Das Gewebe oder Flechtwerk oder Gewirk der bielastischen Rückschicht kann aus Polyethylenterephthalat einer Dicke von 0,01 bis 1,00, insbesondere 0,25 bis 0,75 und insbesondere etwa 0,5 mm bestehen, gemessen jeweils vorzugsweise ungestreckt.

Sofern das erfindungsgemäße Pflaster zusätzlich zur Rückschicht eine Deckschicht aufweist, kann das Material der Deckschicht dasselbe Material sein wie das der Rückschicht und vorzugsweise wirkstoffundurchlässig sein oder es kann dasselbe Material wie das der wiederablösbaren Schutzschicht sein.

Bei dem Material der wiederablösbaren Schutzschicht bzw. Abziehfolie kann es sich um Polyethylen (PE), Polypropylen (PP) oder Polyester handeln, insbesondere Polyethylenterephthalat.

Die wiederablösbare Schutzschicht sollte die Rückschicht allseitig überragen.

### Beispiele 1 bis 2

Es wurden auf ein Band eines einseitig silikonisierten Schutzschicht-Materials (Polyethylenterephthalat; z.B. Primeliner PET 75 mikro m 1S) Pflasterkerne gespendet. Die Pflasterkerne waren ein Verbund aus einer wirkstoffundurchlässigen Trennschicht bzw. Deckschicht (Polyethylenterephthalat; z.B. Primeliner PET 75 mikro m 1S) und aus einer Matrix-Schicht (z.B. DuroTak 6911A). Danach wurde ein Band aus einem bielastischen Rückschicht-Material (Polyethylenterephthalat-Band in Längs- und Querrichtung unelastisch, Dehnung in Richtung der beiden Diagonalen größer gleich 10 % bzw. größer gleich 35 %) auf das Band des Schutzschicht-Materials mit den darauf gespendeten Pflasterkernen aufkaschiert. Aus dem Band des aufkaschierten bielastischen Rückschicht-Materials wurden einzelne Rückschichten derart gestanzt, dass die Rückschichten die Trennschichten mit ihren Matrix-Schichten auf dem Band des Schutzschicht-Materials allseitig abdeckten. Schließlich wurden aus dem Band des Schutzschicht-Materials einzelne Schutzschichten gestanzt, die die Rückschichten allseitig überragten.

Die Distanz der sich gegenüberliegenden Ränder der Rückschichten wichen um weniger als 0,5 % von den zu ihnen parallelen Abschnitten der Kerbe ab, die das Stanzwerkzeug in den Schutzschichten hinterlassen hatte.

### Vergleichbeispiel 1

Es wurde EP 1 009 393 B1 Vergleichsbeispiel 1 nachgearbeitet. Etwa 50 % der hergestellten Pflaster zeigten entweder einen Schüsselbildungseffekt oder die bielastischen Rückschichten hatten sich in Prozessrichtung um mehr als 5 % von der Stanzkerbe zurückgezogen.

### Tests 1 bis 2

Es wurde die Längsdehnung eines Rückschicht-Materials (Firma Karl Otto Braun/Wolfstein; Polyethylenterephthalat; Flächengewicht 130 +/- 20 g/m²; PET-Gewebe; TL 053/11-C) mit einer Zugkraftprüfmaschine (Firma Zwick/Ulm) gemäß DIN 61632 bestimmt.
Maschine Z 0.5
Kraftaufnehmen: XforceP, 500 N, G.-Nr. 450
Teststreifenbreite 30 mm; b null 30 mm
Einspannlänge bei Startposition
   Test 1: 200,06 mm
   Test: 2 199,70 mm
Zielposition im Abschnitt/Belastung 10 N/cm
Geschwindigkeit im Abschnitt 500 mm/min
Haltezeit im Abschnitt 60 s
Vorkraft 10 mn (keine Haltezeit der Vorkraft, Kraft wird bei Erreichen nicht genullt)
Dehnung absolut (Bindenlänge gedehnt - Einspannlänge) 2
   Test 1: 105,97 mm
   Test 2: 104,33 mm
epsilon ges
   Test 1: 52,97 %
   Test 2: 52,24 %

## Patentansprüche

1. Pflaster
(a) mit einer obligatorischen gegebenenfalls haftkleber-beschichteten bielastischen Rückschicht,
(b1) mit einer haftklebenden Matrix-Schicht und einer fakultativen haftklebenden Klebemittel-Schicht oder
(b2) mit einer nicht-haftklebenden Matrix-Schicht und einer haftklebenden Klebemittel-Schicht und
(c) mit einer obligatorischen wiederablösbaren Schutzschicht,
wobei die Bielastizität der bielastischen Rückschicht **dadurch gekennzeichnet ist, dass** die Rückschicht in zwei verschiedenen Richtungen in nicht nur unwesentlichem Umfang elastisches Verhalten zeigt, wobei
- die Rückschicht ein bielastisches Gewebe oder ein bielastisches Flechtwerk (bielastisches Geflecht) oder ein bielastisches Gewirke umfasst oder ist,
- die Bielastizität der Rückschicht **dadurch gekennzeichnet ist, dass** die Rückschicht in zwei verschiedenen Richtungen in nicht nur unwesentlichem Umfang elastisches Verhalten zeigt, wobei
- bei einer Rückschicht mit oder aus einem Gewebe diese beiden voneinander verschiedenen Richtungen weder parallel zur Kette des Gewebes noch in Richtung senkrecht zur Kette verlaufen und sowohl zum Schuss als auch zur Richtung senkrecht dazu schräg oder geneigt sind und
- bei einer Rückschicht mit oder aus einem Flechtwerk diese beiden voneinander verschiedenen Richtungen weder parallel zum Strang des Flechtwerks noch in Richtung senkrecht zum Flechtstrang verlaufen und sowohl zum Flechtstrang als auch zur Richtung senkrecht dazu schräg oder geneigt sind und
- bei einer Rückschicht mit oder aus einem Gewirke die beiden voneinander verschiedenen Richtungen weder parallel zur Richtung der Gewirkefäden noch in dazu senkrechter Richtung verlaufen und sowohl zur Richtung der Gewirkefäden als auch in dazu senkrechter Richtung schräg oder geneigt sind.

2. Pflaster nach Anspruch 1, wobei das Pflaster eine Deckschicht umfasst.

3. Pflaster nach Anspruch 1, bei dem die Elastizität der bielastischen Rückschicht in zwei voneinander verschiedenen Richtungen mindestens 10 % beträgt(gemessen nach DIN 61632).

4. Pflaster nach Anspruch 2, bei dem die Elastizität der bielastischen Rückschicht in mindestens einer der beiden voneinander verschiedenen Richtungen oder in beiden Richtungen 30 bis 56 % beträgt(gemessen nach DIN 61632).

5. Pflaster nach mindestens einem der Ansprüche 1, 2 oder 3, bei dem die bielastische Rückschicht wirkstoffundurchlässig ist.

6. Pflaster nach mindestens einem der Ansprüche 1, 2, 3 oder 4mit einer bielastischen Rückschicht mit oder aus einem Gewebe oder mit oder aus einem Flechtwerk oder mit oder aus einem Gewirke aus Polyethylenterephthalat.

7. Pflaster nach Anspruch 5 mit einer bielastischen Rückschicht, bei der das Polyethylenterephthalat ein Flächengewicht von 40 bis 150 g/m² aufweist.

8. Pflaster nach mindestens einem der Ansprüche 5 und/oder 6 mit einer bielastischen Rückschicht, bei der das Polyethylenterephthalat eine Dicke von 0,01 bis 1,00 mm aufweist, gemessen jeweils vorzugsweise ungestreckt.

## Claims

1. Patch
(a) comprising a mandatory possibly self-adhesive-coated bielastic backing layer,
(b1) comprising a self-adhesive matrix layer and an optionally self-adhesive adhesive layer or
(b2) comprising a non-self-adhesive matrix layer and a self-adhesive adhesive layer and
(c) comprising a mandatory removable protective layer,
whereby the bielasticity of the bielastic backing layer is **characterised in that** the backing layer shows to not an insignificant extent elastic behaviour in two different directions, whereby
- the backing layer comprises or is made of a bielastic fabric or a bielastic interlace (bielastic mesh) or a bielastic knitted fabric,
- the bielasticity of the backing layer is **characterised in that** the backing layer shows to not an insignificant extent elastic behaviour in two different directions, whereby
- with a backing layer comprising or consisting of a fabric these two different directions run neither parallel to the warp of the fabric nor in vertical direction to the warp and are vertically inclined or sloped to both the weft and the direction, and
- with a backing layer comprising or consisting of an interlace these two different directions run neither parallel to the interlace strand nor in vertical direction to the interlace strand and are vertically inclined or sloped to both the interlace strand and the direction, and
- with a backing layer comprising or consisting of a knitted fabric the two different directions run neither parallel to the direction of the threads of the knitted fabric nor in vertical direction thereto and are inclined or sloped to both the direction of the threads of the knitted fabric and in vertical direction thereto.

2. Patch according to claim 1, whereby the patch comprises a top layer.

3. Patch according to claim 1, whereby the elasticity of the bielastic backing layer in two different directions is at least 10 % (measured according to DIN 61632).

4. Patch according to claim 2, whereby the elasticity of the bielastic backing layer in at least one of the two different directions or in both directions is 30 to 56 % (measured according to DIN 61632).

5. Patch according to at least one of claims 1, 2 or 3, whereby the bielastic backing layer is impermeable to active substance.

6. Patch according to at least one of claims 1, 2, 3 or 4 comprising a bielastic backing layer comprising or consisting of a fabric or comprising or consisting of an interlace or comprising or consisting of a weave of polyethylene terephthalate.

7. Patch according to claim 5 comprising a bielastic backing layer, whereby the polyethylene terephthalate has a surface weight of 40 to 150 g/m².

8. Patch according to at least one of claims 5 and/or 6 comprising a bielastic backing layer whereby the polyethylene terephthalate has a thickness of 0.01 to 1.00 mn, in each case preferably measured unstretched.

## Revendications

1. Emplâtre
(a) présentant une couche dorsale biélastique obligatoire le cas échéant enduite d'un agent auto-adhésif,
(b1) comprenant une couche matrice adhésive et une couche d'agent auto-adhésif facultative adhésive ou
(b1) présentant une couche matrice non-adhésive et une couche d'agent auto-adhésif adhésive et
(c) une couche de protection obligatoire pelliculable,
la biélasticité de la couche dorsale biélastique étant **caractérisée en ce que** la couche dorsale présente, dans une mesure pas seulement négligeable, un comportement élastique dans deux directions différentes,
- la couche dorsale comprenant ou étant soit un tissu biélastique soit un entrelacs biélastique (treillis biélastique) soit un tricot biélastique,
- la biélasticité de la couche dorsale étant **caractérisée en ce que** la couche dorsale présente, dans une mesure pas seulement négligeable, un comportement élastique dans deux directions différentes,
- ces deux directions différentes, dans une couche dorsale comprenant ou consistant en un tissu, ne sont ni parallèles à la chaîne du tissu ni en orientation verticalement par rapport à la chaîne et s'étendant de façon diagonale ou inclinée par rapport et à la trame et à la direction verticalement par rapport à celle-ci et
- ces deux directions différentes, dans une couche dorsale comprenant ou consistant en un entrelacs, ne s'étendent ni parallèlement au brin de l'entrelacs ni en orientation verticalement au brin de treillis et de façon diagonale ou inclinée par rapport et au brin de treillis et à la direction verticalement par rapport à celle-ci et
- les deux directions différentes, dans une couche dorsale comprenant ou consistant en un tricot, ne s'étendent ni parallèlement à la direction des fils de tricot ni en direction verticale par rapport à ceux-ci et de façon diagonale ou inclinée par rapport et à l'orientation des fils de tricot et en direction verticale à celle-ci.

2. Emplâtre selon la revendication 1, dans lequel l'emplâtre comprend une couche supérieure.

3. Emplâtre selon la revendication 1, dans lequel l'élasticité de la couche dorsale biélastique totalise, dans deux directions différentes l'une de l'autre, au moins 10% (mesurée selon DIN 61632).

4. Emplâtre selon la revendication 2, dans lequel l'élasticité de la couche dorsale biélastique représente, dans l'une au moins des deux directions différentes l'une de l'autre, 30 à 56% (mesurée selon DIN 61632).

5. Emplâtre selon l'une au moins des revendications 1, 2 ou 3, dans lequel la couche dorsale biélastique est imperméable aux agents.

6. Emplâtre selon l'une au moins des revendications 1, 2, 3 ou 4 comprenant une couche dorsale biélastique comprenant ou consistant soit en un tissu soit en un treillis soit en un tricot en polyéthylène téréphtalate.

7. Emplâtre selon la revendication 5 comprenant une couche dorsale biélastique, dans laquelle le polyéthylène téréphtalate présente un grammage compris entre 40 et 150 g/m².

8. Emplâtre selon l'une au moins des revendications 5 et/ou 6 comprenant une couche dorsale biélastique, dans laquelle le polyéthylène téréphtalate présente une épaisseur comprise entre 0,01 et 1,00 mm, de préférence mesurée respectivement en état non étiré.
